# EUROPEAN PATENT SPECIFICATION

(11) **EP 0 728 488 B1**
(45) Date of publication and mention of the grant of the patent: **23.10.2002**
(21) Application number: 96102509.5
(22) Date of filing: 20.02.1996
(51) Int. Cl.: A61M 1/10

(54) **A bag and a pumping unit, especially for cardiac assistance devices**
Sack mit Pumpeneinheit, insbesondere für Herzunterstützungsvorrichtungen
Pompe à poche, en particulier pour dispositifs d'assistance cardiaque

(30) Priority: 23.02.1995 IT TO950133
(43) Date of publication of application: 28.08.1996
(73) Proprietor: MINISTERO DELL' UNIVERSITA' E DELLA RICERCA SCIENTIFICA E TECNOLOGICA, 00144 Roma (IT)
(72) Inventor: Zagara, Maurizio, 00127 Roma (IT); Arabia, Maurizio, 00137 Roma (IT); Mambrito, Bruno, 00044 Frascati (Roma) (IT); Ferri, Enrico, 00175 Roma (IT); Chieco, Silvia, 00100 Roma (IT); Giannitrapani, Marco, 56010 Ghezzano (Pisa) (IT)
(74) Representative: Bosotti, Luciano

(56) References cited:
- EP-A- 0 478 499
- US-A- 4 222 127

## Description

The present invention relates to bags and to pumping units for pumps. The invention has been developed with particular attention to its possible use in the production of pumps for cardiac assistance devices (artificial ventricles, artificial hearts, etc.). The scope of the invention is not intended to be limited to this specific field of application, however, but extends to the pumping of fluids in general, principally with regard to the pumping of fluids in the biomedical field.

In this context, use is currently made of pumps each comprising a compressible pumping element in the form of a bag and a thrust element (a plate) which acts on the bag in order to compress it cyclically, the bag being housed in a respective shell.

A pump of this type may be operated by a rotary motor with an associated screw which converts the movement of the rotor of the motor into the translational movement of the plate.

Exemplary of such prior art is the arrangement shown in EP-A- 0 478 499, after which the preamble of Claim 1 was patterned. Also US-A-4 222 127 is of general interest for the invention.

In producing pumping devices of this type it is necessary to take account of various factors.

In the first place, it is desirable to ensure that the bag-like element can be used with great efficiency and hence with the ability to change from a fully-extended volume to a fully-compressed volume which is virtually zero, without the formation of areas in which the pumped fluid (for example, blood) is stagnant and/or spaces which are not used for the purposes of the pumping action. This is also desirable on account of the fact that any stagnant areas of the blood inside the bag may give rise to thromboses.

Moreover, it is desirable to ensure that the bag is not subject to inappropriate structural stresses which might cause premature rupture, such as stresses which may result from the formation of more or less irregular pleats during the change from the extended condition to the compressed or crushed condition. This factor becomes of decisive importance in particular in applications in which the pump has to be implanted in a patient's body in conditions in which any maintenance or replacement operation is in any case considered highly undesirable.

It is also desirable to achieve an optimal situation in terms of the relationship between the efficiency of the pump and its overall size. Although this requirement is important for many applications, it becomes of primary importance in the case of pumps for implantation in a patient's body, for example, in a thoracic or abdominal position, particularly with regard to possible implantation in small patients. In that respect, US-A-4 557 673 discloses a chamber structure for an implantable blood pump including inlet and outlet conduits associated with ports whose shape is substantially constant during pump operation.

The object of the present invention is to provide a bag and, in a further aspect, a pumping unit, for pumps which can satisfy the requirements expressed above in an excellent manner, overcoming the intrinsic problems of the solutions of the prior art.

According to the present invention, this object is achieved by virtue of a bag and of a drive unit for pumps having the specific characteristics recited in the following claims.

The invention will now be described, purely by way of non-limiting example, with reference to the appended drawings, in which:
Figures 1 and 2 show, in theoretical diametral section, a pump formed according to the invention, seen in two different operating positions,

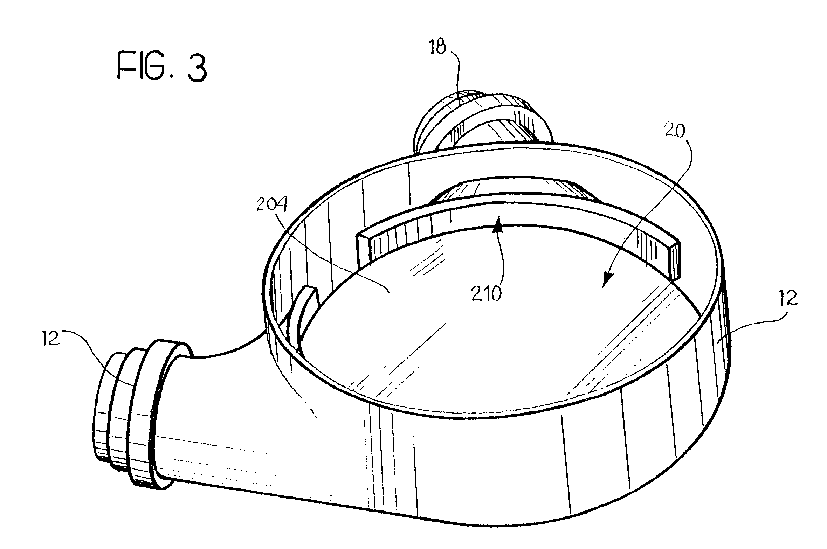

Figure 3 shows part of a pump according to the invention in the assembled condition, and
Figure 4 is an exploded view of the components of the part shown in Figure 3.

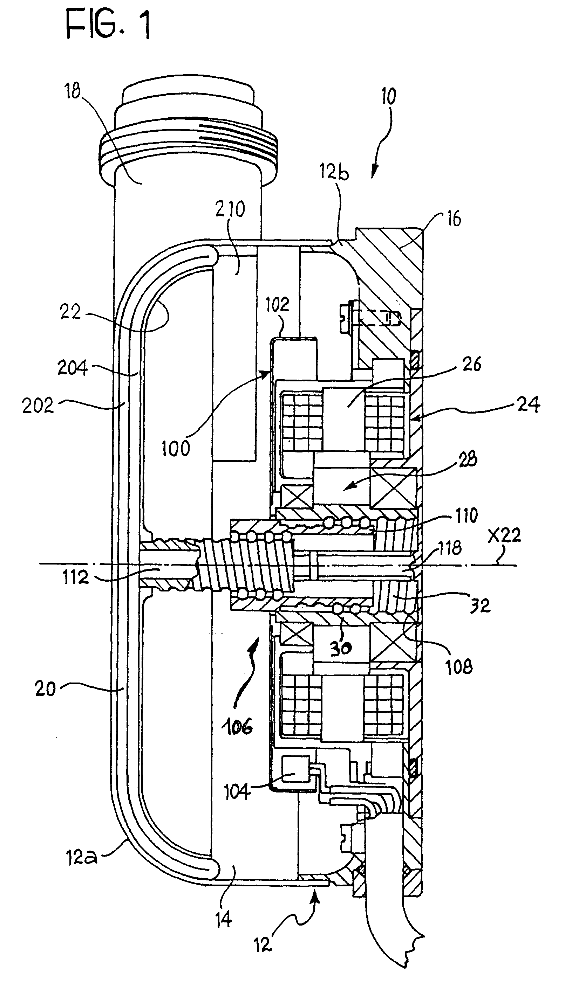

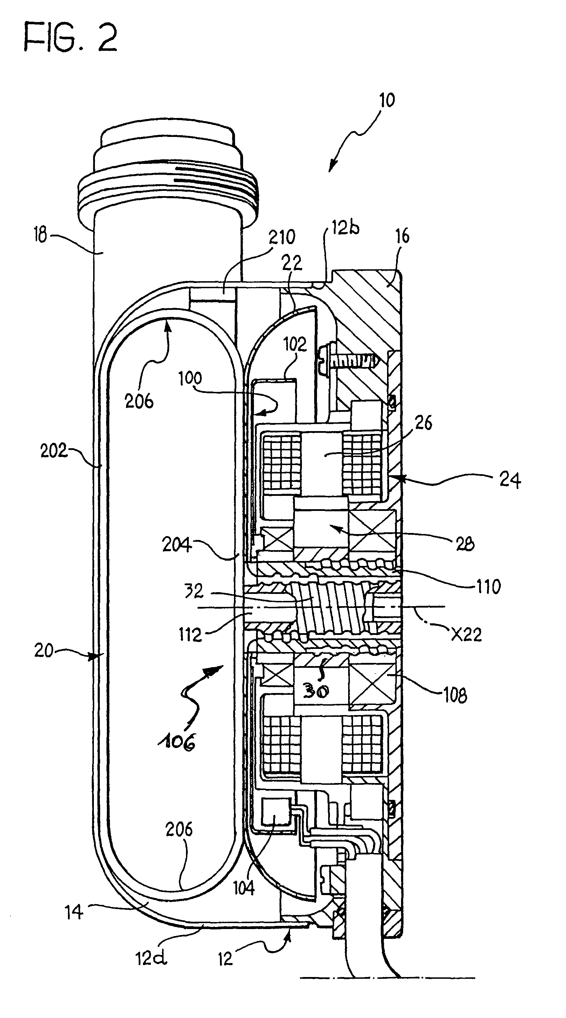

In Figures 1 and 2, as an example of a pump, a cardiac assistance device (VAD) constituted specifically by a so-called artificial ventricle is generally indicated 10. The device 10 is housed in a casing or shell 12 of material compatible with the requirements for implantation in the body (for example, titanium).

Essentially two components or sections can be distinguished within the device 10, that is, a pumping section or unit 14 and an actuating section 16 comprising an electromechanical actuator which drives the pumping unit 14.

The latter is constituted essentially by a cup-shaped portion 12a of the shell 12 having connectors 18 (only one of which is visible in Figures 1 and 2, which correspond, as stated, to a diametral section of the casing of the device 10 which is approximately cylindrical in shape) for connection to the blood suction and delivery lines. The shell portion 12a houses a flexible bag 20 which is intended in use to be connected sealingly to the blood lines connected to the device 10, usually with the interposition of valves (not shown) which regulate the blood flow through the blood lines and the device 10 so as to achieve a pulsed one-way flow. The bag 20 is usually made of a flexible material such as, for example, polyurethane, having biocompatibility (haemocompatibility) characteristics.

According to known criteria, the pumping action takes place as a result of the cyclic compression of the bag 20 brought about by means of a thrust member such as a plate 22 movable to and fro along a respective central axis X22. In practice, the bag 20 is interposed between the base of the cup-shaped portion 12a of the shell 12 and the plate 22.

The reciprocating movement of the plate 22 relative to the base of the cup-shaped portion 12 brings about cyclically:
- compression of the bag as a result of the movement of the plate 22 towards the base of the cup-shaped portion 12a (Figure 1) with consequent expulsion of blood from the device 10 through the delivery line, and
- expansion of the bag as a result of the return of the plate 22 to the position in which is it spaced from the base of the cup-shaped portion 12a (Figure 2) consequently permitting the blood to reach the interior of the bag 20 through the suction line.

The reciprocating movement of the plate 22 along the axis X22 (which usually corresponds to the central axis of the cup-shaped portion 12a) takes place under the action of a motor, typically constituted by an electric motor 24. This is usually a brushless motor having an approximately disc-like shape and composed of an annular outer stator 26 which is fitted in the mouth portion of the portion 12b of the shell 12 which encloses the actuating section 16, and of a central rotor 28.

A motor such as the motor 24 is available commercially and produced by Inland Motors, a division of the Kollmorgen Corporation (Illinois, United States).

The rotor 28 incorporates in a central position, that is, in alignment with the axis X22, (or is itself configured in the form of) a lead screw 30 with which a screw 32 is engaged. This is preferably a ball-bearing screw 32 of which the end facing and projecting towards the pumping section 14 is fixed to the plate 22.

According to a particularly preferred embodiment (and based on the solution which forms the specific subject of EP-A-0 728 489 the screw 32 is a screw with a telescopic structure comprising:
- a cylindrical lead screw 108 for fitting (usually with interference) inside the rotor 28 of the motor 24 so as to be fixed for rotation therewith,
- an intermediate cylindrical sleeve 110 the outer surface of which has the characteristics of a screw engaged with the lead screw 108 and the inner surface of which has the characteristics of a further lead screw of smaller diameter than the lead screw 108, and
- an inner element 112 the outer surface of which has the characteristics of a screw engaged with the internal thread (lead screw) of the sleeve 110; the inner element 112 supports the plate 22 in a central position.

The plate 22 is restrained against rotation which may be induced by the motor 24 by the presence of a pin 118 which extends from the shell portion 12b towards the interior of the shell 12 in alignment with the axis X22, the pin 118 having a prismatic cross-section complementary with the cross-section of an axial hole in the inner element 112 of the screw 32.

For further structural details of the motor 24 and of the members connected thereto, reference may usefully be made to the description of the application mentioned above; however, this further technical information is not essential *per se* for the purposes of the understanding and implementation of the present invention.

As already stated above, the cyclic pumping of the blood by the device 10 takes place by means of a reciprocating translational movement of the plate 22 along the axis X22. This movement is brought about by the rotation of the motor 24 alternately in the two senses. The rotary movement of the motor 24 and the resulting translational movement of the plate 22 are also associated with a cyclic contraction and extension of the screw 32, owing to its telescopic structure.

In particular, the most retracted position of the plate 22 in which it is close to the motor 24 corresponds to the arrangement of the screw 106 in the most axially contracted condition shown in Figure 2; in this situation, the inner element 112 is completely, or almost completely, housed in the sleeve 110 which in turn is almost completely housed in the lead screw 108, the axial dimensions of which are determined so as to correspond essentially to the axial thickness of the motor 24.

When this condition, which corresponds to the maximum possible extension permitted for the bag 20, has been reached, a change in the sense of rotation of the motor 24 brings about:
- on the one hand, axial translation of the sleeve 110 relative to the lead screw 108 with a consequent forward movement towards the extended position shown in Figure 1, and
- on the other hand, axial translation of the inner element 112 relative to the sleeve 110 with a consequent completion of the extension movement of the screw.

The position shown in Figure 1, which corresponds to the most squashed condition of the bag 20 is thus reached.

If the sense of rotation of the motor 24 is changed again the plate can then be returned backwards towards the motor 24, returning the screw 116 to the contracted condition shown in Figure 2.

It can also be noted that the motor 24 has a stator 26 with generally smaller radial dimensions than the shell 12 and, more specifically, than the plate 22.

This enables a rotor element 100 to be keyed to the rotor 28 of the motor 24, the rotor element 100 having a skirt portion 102 with locally differentiated surface characteristics, for example, owing to the presence of notches, which extends around the periphery of the stator 26 forming an annular space in which electro-optical sensors (detector unit) 104 are located. This is in order to form (according to a known solution) a so-called optical encoder the function of which is to detect the rotation parameters of the motor 24 (particularly the position reached by the motor 24 during rotation) so as to be able to obtain an incremental position reference during the detection of the movement of the plate 22.

As can best be seen in the view of Figure 2, the location of the element 100 in a position at least partially surrounding the stator 26 of the motor 24 and the selection of radial dimensions smaller than those of the plate 22 which has a generally cup-shaped configuration with its cavity facing towards the motor 24, means that, in the most retracted position of Figure 2, the plate 22 can be retracted until its base surface is practically in contact with the corresponding surface of the element 100. This enables optimal use to be made of the relative movement space, consequently minimizing the axial dimensions of the device 10 as a whole.

As can be appreciated from a comparison of Figures 1 and 2, the bag 20 is constructed (with the use of widely known materials and techniques which do not need to be described in detail herein) in the form of an element with a main disc-shaped body (an internal pumping volume) comprising, in the extended condition (Figure 2), two opposed faces 202, 204 preferably with circular profiles, connected by an edge portion 206. Observed in a theoretical diametral section of the bag 20 such as the section plane of Figures 1 and 2, the edge 206 has a substantially circular profile, that is, a profile in which two symmetrical, complementary portions can be distinguished, each corresponding approximately to a quarter of a circle.

The overall effect of this shape of the bag 20 can readily be appreciated from the view of Figure 1 which shows how, by virtue of the complementary shape of the plate 22 and of the base portion of the cup-shaped portion 12a of the shell 12, it is possible to ensure that the portion of the bag 20 which faces the plate 22 and is exposed to the action thereof, starting from the extended condition of the bag 20 (Figure 2) in which this portion (the base 204 and the corresponding half of the edge 206 which surrounds it) has a generally bowl-shaped configuration, can reach the compressed configuration of Figure 1 in which the said portion of the bag 20 is turned inside out and adopts a configuration complementary to itself and hence to the remaining portion of the bag 20 (the base 202 and the corresponding half of the edge 206 which surrounds it) which is in contact with the cup-shaped portion 12a. This ensures (in the compressed condition) that the volume inside the bag 20 is reduced almost to zero.

The change from the extended position of Figure 2 to the compressed position of Figure 1 is thus achieved whilst uneven stresses such as to lead to the formation of pleats, folds or regions subject to stress in general which, particularly if repeated, could lead to damage of the material constituting the bag 20 are prevented.

Although the selection of a circular profile for the peripheral edge of the bag is to be considered preferable for the purposes of structural simplicity and efficiency of operation, the same general principle may also be used in bags of different shapes, for example, bags in which the profile of the peripheral edge (observed in a diametral plane relative to the bag) has, for example, a parabolic, elliptical, or mixtilinear shape, provided that the portion of the bag 20 which is exposed to the action of the plate 22 has a shape such that it can be "turned inside out to a complementary position" that is, this portion can be brought to lie, with form coupling, inside the other portion of the bag which is in contact with the cup-shaped portion 12a of the shell 12.

It can easily be appreciated that the result described above in the changing of the bag from the extended condition to the compressed condition is brought about under optimal conditions when both the plate 22 and the cup-shaped portion 12a of the shell 12 (and principally the base portion of the cup-shaped portion) have exactly complementary cup shapes (for example, in the embodiment shown, with a flat circular base surrounded by an edge with a quarter-circular profile) so as to define the shape to which the portion of the bag which is subjected to the action of the plate 22 is turned inside out to a complementary position inside the remaining portion of the bag.

At least in principle, the result just described could also be achieved if the portion of the bag 20 which is intended to be brought into contact with the shell 12 (the portion including the base wall 202) were made of a more or less rigid material, or in any case a more rigid material than the rest of the bag 20 which has to be turned inside out in order to change from the extended position to the compressed position under the action of the plate 22. In particular, although not specifically shown in the drawings, this rigid portion of the bag could even be wholly or partly replaced by, and thus merged with, the base of the cup-shaped portion 12a of the shell 12, possibly including the connectors 18 connected thereto.

It can be seen from the foregoing that the generally disc-like configuration imparted to the bag 20 enables the base of the cup-shaped portion 12a to be made substantially level with the connectors 18 which, in contrast with what usually occurs in solutions according to the prior art, do not project at all, even locally, from the imaginary plane in which the wall or base is situated.

This, in cooperation with the cup-like shape of the plate 22 with radial dimensions such as to surround a good part of the stator 26 in the motor 24, and the telescopic configuration of the screw 32, permits a considerable reduction in the length of the device 10 measured in an axial direction (that is, along the axis X22), in fact reducing it to a maximum dimension corresponding approximately to twice the axial thickness of the motor 24. This reduction of the axial dimensions is particularly advantageous in the case of pumping devices 10 for implantation in the human body.

Moreover, it will be noted from an observation of Figures 1 and 2 that the reduction in the thickness of the bag 20 enables this thickness to be brought down to a value approximately comparable with the diameters of the suction and delivery lines 18.

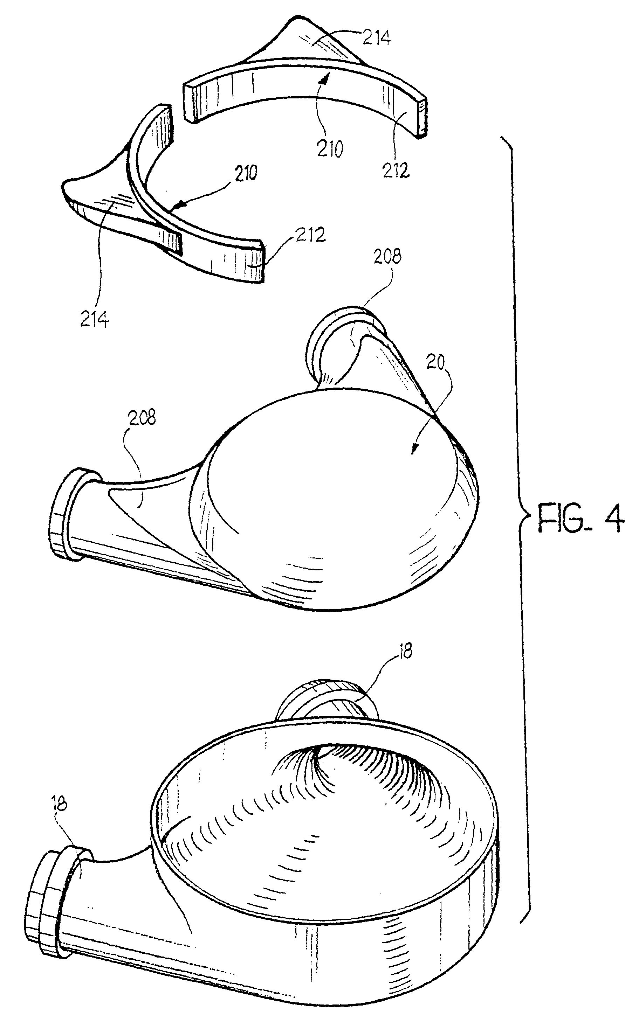

Since the bag 20 is virtually completely crushed (Figure 1), in the solution according to the invention, it is important to ensure that, when the bag 20 is crushed, there is no stenosis or obstruction of the lines 18 particularly of the extensions of the bag 20 (the extensions indicated 208 in Figure 4) which, in the device 10 assembled, are intended to occupy the openings of the connectors 18 in the shell 12. In the solution shown, this result is achieved by conferring on the extensions 208 a shape which gradually becomes more crushed as it approaches the central body of the bag 20.

This crushing is brought about according to a principle of the maintenance of a constant cross-sectional area: if the net desired cross-sectional area of the extensions 208 is defined at their distal, outer ends relative to the bag 20, the heights of the extensions, measured in the direction in which the portions 202, 204 face one another and hence in the direction of movement of the plate 22 are gradually reduced, the lines simultaneously being widened in a circumferential direction relative to the body of the bag 20; this takes place whilst a constant net value of the cross-sectional areas of the lines 208 is maintained. The heights of the extensions 208 measured in the direction in which the bag is crushed and their widths measured circumferentially relative to the body of the bag 20 thus increase and decrease, respectively, in a complementary manner, in the direction away from the body of the bag 20.

To ensure that the extensions 208 are restrained correctly when they are subjected to the internal pressure of the blood which passes into them, it is advisable to have a substantially complementary flattened configuration in the corresponding regions (the connectors 18) of the shell portion 12a, thus also maintaining an almost constant cross-sectional area from the root portion towards the end portions, in this case. A similar configuration with heights of the internal cavities of the connectors 18 increasing gradually from their proximal portions towards their distal portions with a corresponding reduction in width in a circumferential direction relative to the body of the shell portion 12a is achieved by the location of a respective insert 210 inside each connector 18. Each insert 210 comprises a base portion 212 which is intended to extend inside the cup-shaped portion 12a so as to ensure that the continuity of its peripheral wall is substantially maintained, as well as a projecting portion 214 which can be defined as approximately "fin-shaped" and which is intended to extend into the respective connector 18 so as to form a partial obstruction of its opening, the extent of the obstruction decreasing gradually towards the distal end of the connector 18. This achieves, in a complementary manner, the necessary gradual increase in height and gradual reduction in width of the extension 208 which is intended to extend inside the respective connector 18.

## Claims

1. A drive unit and a bag for pumps comprising respective suction and delivery lines (208) and at least one portion (204, 206) which can be compressed between a thrust element (22) and a cup shaped portion (12a), wherein the bag is constructed in the form of an element with a main disc-shaped body defining a generally disc-shaped internal pumping volume thus having a direction in which the bag is crushed and the at least one compressible portions (204, 206) is selectively deformable between an extended condition in which it has a configuration generally in the form of a bowl, and a compressed condition in which the portion is turned inside out to a complementary position so as to reduce the internal pumping volume substantially to zero, the drive unit comprising the thrust element (22), as well as means (24, 32) for driving the thrust element, the means being able to impart to the thrust element (22) a reciprocating movement along a predetermined axis (X22) relative to the cup shaped portion (12a) with the compressible portion (204, 208) interposed between the thrust element (22) and the cup shaped portion (12a), the thrust element (22) having a profile which is substantially identical in a complementary manner to the configuration adopted by the compressible portion of the bag (20) in the extended condition so that, in the compressed condition, the thrust element (22) compresses the compressible portion of the bag (20) to a condition of substantial form coupling with the cup shaped portion (12a); the drive unit comprising a shell (12) with a base of which defining the cup shaped portion (12a), respective connectors (18) being associated with the shell for receiving the supply and delivery lines (208) of the bag, the connectors (18) branching out from the shell (12) substantially without projecting axially from said cup shaped portion (12), **characterised in that** said drive unit comprises inserts (210) associated with the connectors (18) to partially block the connectors (18) in order to confine the suction and delivery lines (208) in spaces inside the connectors (18) having heights, measured in the direction in which the bag is crushed as a result of movement of the thrust element (22), and widths, measured in a circumferential direction relative to said disc-shaped body and the cup shaped portion (12a), which increase and decrease, respectively, in the direction away from the cup shaped portion (12a), whereby said suction and delivery lines (208) have uniform cross-sectional areas.

2. The device according to Claim 1, **characterized in that** each of the inserts (210) comprises a curved portion (212) which can be located on a peripheral cup shaped portion of the shell (12) so as to maintain substantial continuity of the cup shaped portion which is in contact with the bag (20) and a projecting portion (214) which extends into the respective connector (18) in use.

3. The device according to Claim 1, **characterized in that** the pumping volume has an outer edge (206) which, when observed in a radial plane relative to the volume, has a substantially circular profile.

4. The device according to any one of the preceding claims, **characterized in that** the bag (20) is formed integrally of a flexible material.

5. The device according to Claim 4, **characterized in that** the flexible material is a biocompatible material.

6. The device according to Claim 1, **characterized in that** the suction and delivery lines (208) are formed integrally with the remaining portions (202, 204, 208) of the bag (20).

7. The device according to any one of the preceding claims, **characterized in that** the bag (20) has a generally disc-like shape with a generally circular body.

8. The device according to Claim 1, **characterized in that** the thrust element (22) is constituted essentially by a cup-shaped body having a peripheral edge with a substantially circular profile.

9. The device according to Claim 8, **characterized in that** the driving means (24, 32) comprise a generally disc-shaped motor (24) with a rotor portion (28) rotatable about a respective principal axis (X22) with drive-conversion means (32) acting between the motor (24) and the thrust element (22) substantially on the principal axis (X22).

10. The device ccording to Claim 9, **characterized in that** the motor (24) has smaller radial dimensions than the thrust element (22) so that, in a most retracted position relative to the motor (24), the edge of the thrust element (22) at least partially surrounds the motor (24).

11. The device according to Claim 10, **characterized in that** a position detector unit (100, 102, 104) associated with the motor (24) comprises a rotor element (100) with a skirt portion (102) which surrounds the motor (24) and has smaller diametral dimensions than the thrust element (22) so that, in the most retracted position, the thrust element houses the rotor element (100).

## Patentansprüche

1. Eine Antriebseinheit und eine Tasche für Pumpen, mit entsprechenden Ansaug- und Zuführleitungen (208) und mit zumindest einem Bereich (204, 206), welcher zwischen einem Stoßelement (22) und einem tassenförmigen Bereich (12a) komprimierbar ist, wobei die Tasche in Form eines Elements mit einem scheibenförmigen Hauptkörper ausgebildet ist, welcher ein allgemein scheibenförmiges inneres Pumpvolumen definiert und somit eine Richtung erfährt, in welcher die Tasche zusammengedrückt wird, und wobei der zumindest eine komprimierbare Bereich (204, 206) wahlweise zwischen einem ausgedehnten Zustand, in welcher er allgemein die Gestalt einer Schüssel aufweist, und einem komprimierten Zustand, in welchem der Bereich zu einem komplementären Zustand umgestülpt ist, um das interne Pumpvolumen im wesentlichen auf null zu reduzieren, deformierbar ist,
wobei die Antriebseinheit das Stoßelement (22) sowie auch ein Mittel (24, 32) zum Antrieb des Stoßelements aufweist, wobei das Mittel in der Lage ist, das Stoßelement (22) in eine Hin- und Herbewegung entlang einer vorbestimmten Achse (X22) zu versetzen, welche relativ zu dem tassenförmigen Bereich (12a) erfolgt, wobei der komprimierbare Bereich (204, 206) zwischen dem Stoßelement (22) und dem tassenförmigen Bereich (12a) angeordnet ist, und wobei das Stoßelement (22) ein Profil aufweist, welches in komplementärer Weise im wesentlichen identisch mit der Form ist, welche der komprimierbare Bereich der Tasche (20) in der ausgedehnten Stellung einnimmt, so daß das Stoßelement (22) in der komprimierten Stellung den komprimierbaren Bereich der Tasche (20) in einen Zustand zusammendrückt, in welchem dieser im wesentlichen eine dem tassenförmigen Bereich (12a) entsprechende Form annimmt; die Antriebseinheit ein Gehäuse (12) mit einer Basis aufweist, die den tassenförmigen Bereich (12a) definiert, wobei dem Gehäuse Anschlüsse (18) für die Aufnahme der Zuführ- und Ablaßleitungen (208) der Tasche zugeordnet sind und die Anschlüsse (18) von dem Gehäuse (12) abzweigen, ohne dabei wesentlich axial von dem genannten tassenförmigen Bereich vorzustehen, **dadurch gekennzeichnet, daß** die genannte Antriebseinheit Einsätze (210) aufweist, welche den Anschlüssen (18) zugeordnet sind, und die die Anschlüsse (18) teilweise blockieren, um die Ansaugund Zuführleitungen (208) in Räumen innerhalb der Anschlüsse (18) zu begrenzen, die in der Richtung, in welcher die Tasche als Resultat der Bewegung des Stoßelements (22) zusammengedrückt wird, Höhen aufweisen, und in Umfangsrichtung des genannten scheibenförmigen Körpers Breiten aufweisen, die in der Richtung weg vom tassenförmigen Bereich (12a) zunehmen bzw. abnehmen, wobei die genannten Ansaug- und Zuführleitungen (208) einheitliche Querschnittsflächen aufweisen.

2. Das Gerät gemäß Anspruch 1, **dadurch gekennzeichnet, daß** jeder der Einsätze (210) einen gewölbten Bereich (212) aufweist, welcher sich an einem tassenförmigen Umfangsbereich des Gehäuses (12) befinden kann, um die Kontinuität des tassenförmigen Bereichs im wesentlichen aufrecht zu erhalten, der in Kontakt mit der Tasche (20) und einem vorgelagerten Bereich (214) steht, welcher sich beim Gebrauch in den entsprechenden Anschluß (18) erstreckt.

3. Das Gerät gemäß Anspruch 1, **dadurch gekennzeichnet, daß** das Pumpvolumen eine äußere Kante (206) aufweist, welche in einer radialen Ebene relativ zu dem Volumen betrachtet, ein im wesentlichen rundliches Profil aufweist.

4. Das Gerät nach einem der vorhergehenden Ansprüche, **dadurch gekennzeichnet, daß** die Tasche (20) vollständig aus einem elastischen Material ausgebildet ist.

5. Das Gerät nach Anspruch 4, **dadurch gekennzeichnet, daß** das elastische Material ein biokompatibles Material ist.

6. Das Gerät nach Anspruch 1, **dadurch gekennzeichnet, daß** die Ansaug- und Zuführleitungen (208) und die verbleibenden Bereiche (202, 204, 208) der Tasche (20) in einem Stück ausgebildet sind.

7. Das Gerät gemäß einem der vorhergehenden Ansprüche, **dadurch gekennzeichnet, daß** die Tasche (20) eine allgemeine scheibenförmige Form mit einem im allgemeinen rundlichen Körper aufweist.

8. Das Gerät gemäß Anspruch 1, **dadurch gekennzeichnet, daß** das Element (22) im wesentlichen durch einen tassenförmigen Körper gebildet wird, welcher eine äußere Kante mit einem im wesentlichen rundlichen Profil aufweist.

9. Das Gerät gemäß Anspruch 8, **dadurch gekennzeichnet, daß** das Antriebsmittel (24, 32) einen im wesentlichen scheibenförmigen Motor (24) aufweist, welcher einen Rotorbereich (28) besitzt, der um eine entsprechende Hauptachse (X22) rotierbar ist, wobei der Rotorbereich (28) ein Antriebsübersetzungsmittel (32) aufweist, welches zwischen dem Motor (24) und dem Stoßelement (22) im wesentlichen auf der Hauptachse (X22) arbeitet.

10. Das Gerät gemäß Abspruch 9, **dadurch gekennzeichnet, daß** der Motor (24) kleinere radiale Dimensionen als das Stoßelement (22) aufweist, so daß in der am weitesten zurückgezogenen Position, relativ zu dem Motor (24), die Kante des Stoßelements (22) zumindest teilweise den Motor (24) umschließt.

11. Das Gerät gemäß Anspruch 10, **dadurch gekennzeichnet, daß** eine Positionsdetektionseinheit (100, 102, 104) mit dem Motor (24) verbunden ist, welche ein Rotorelement (100) mit einem Randbereich (102) enthält, der den Motor (24) umgibt und kleinere diametrale Dimensionen aufweist als das Stoßelement (22), so daß in der am meisten zurückgezogenen Position das Stoßelement das Rotorelement (100) umschließt.

## Revendications

1. Unité d'entraînement et poche pour des pompes comprenant des conduits respectifs de succion et de fourniture (208) et au moins une partie (204, 206) qui peut être comprimée entre un élément poussoir (22) et une partie en forme de coupelle (12a), dans laquelle la poche et construite sous la forme d'un élément avec un corps principal en forme de disque définissant un volume de pompage interne généralement en forme de disque et ayant ainsi une direction dans laquelle la poche est écrasée, et ladite au moins une partie compressible (204, 206) est sélectivement déformable entre une condition déployée dans laquelle elle a une configuration généralement en forme de bol, et une condition comprimée dans laquelle ladite partie est retournée vers l'intérieur vers une position complémentaire de manière à réduire sensiblement à zéro le volume de pompage interne,
l'unité d'entraînement comprenant l'élément poussoir (22) ainsi que des moyens (24, 32) pour entraîner l'élément poussoir, ces moyens étant capables d'imposer à l'élément poussoir (22) un mouvement de va-et-vient le long d'un axe prédéterminé (X22) par rapport à la partie en forme de coupelle (12a) avec la partie compressible (204, 208) interposée entre l'élément poussoir (22) et la partie en forme de coupelle (12a), l'élément poussoir (22) ayant un profil qui est sensiblement identique et de manière complémentaire à la configuration adoptée par la partie compressible de la poche (20) dans la condition déployée, de sorte que, dans la condition comprimée, l'élément poussoir (22) comprime la partie compressible de la poche (20) sensiblement jusqu'à une condition d'accouplement en forme avec la partie en forme de coupelle (12a) ; l'unité d'entraînement comprenant une coque (12) avec une base qui définit la partie en forme de coupelle (12a), des connecteurs respectifs (18) étant associés à la coque afin de recevoir les conduits d'alimentation et de fourniture (208) de la poche, les connecteurs (18) étant ramifiés depuis la coque (12) sans se projeter sensiblement axialement depuis ladite partie en forme de coupelle (12), **caractérisée en ce que** ladite unité d'entraînement comprend des inserts (210) associés au connecteur (18) pour bloquer partiellement les connecteurs (18) afin de confiner les conduits de succion et de fourniture (208) dans des espaces à l'intérieur des connecteurs (18) avec des hauteurs, mesurées dans la direction dans laquelle la poche est écrasée en résultat du mouvement de l'élément poussoir (22), et des largeurs, mesurées dans une direction circonférentielle par rapport audit corps en forme de disque et par rapport à la partie en forme de coupelle (12a), qui augmentent et qui diminuent, respectivement, dans la direction en éloignement de la partie en forme de coupelle (12a), grâce à quoi lesdits conduits de succion et de fourniture (208) présentent des aires de section transversale uniformes.

2. Unité selon la revendication 1, **caractérisée en ce que** chacun des inserts (210) comprend une partie incurvée (212) qui peut être située sur une partie périphérique en forme de coupelle de la coque (12) de manière à maintenir sensiblement une continuité de la partie en forme de coupelle qui est en contact avec la poche (20) et d'une partie en projection (214) qui s'étend jusque dans le connecteur respectif (18) en utilisation.

3. Unité selon la revendication 1, **caractérisée en ce que** le volume de pompage a un bord extérieur (206) qui, lorsqu'on l'observe dans un plan radial par rapport au volume, a un profil sensiblement circulaire.

4. Unité selon l'une quelconque des revendications précédentes, **caractérisée en ce que** la poche (20) est formée de manière intégrale en un matériau souple.

5. Unité selon la revendication 4, **caractérisée en ce que** le matériau souple est un matériau biocompatible.

6. Unité selon la revendication 1, **caractérisée en ce que** les conduits de succion et de fourniture (208) sont formés de manière intégrale avec les parties restantes (202, 204, 208) de la poche (20).

7. Unité selon l'une quelconque des revendications précédentes, **caractérisée en ce que** la poche (20) a une forme généralement analogue à un disque avec un corps généralement circulaire.

8. Unité selon la revendication 1, **caractérisée en ce que** l'élément poussoir (22) est essentiellement constitué par un corps en forme de coupelle ayant un bord périphérique avec un profil sensiblement circulaire.

9. Unité selon la revendication 8, **caractérisée en ce que** les moyens d'entraînement (24, 32) comprennent un moteur généralement en forme de disque (24) avec une partie de rotor (28) en rotation autour d'un axe principal respectif (X22) avec des moyens de conversion d'entraînement (32) agissant entre le moteur (24) et l'élément poussoir (22) sensiblement sur l'axe principal (X22).

10. Unité selon la revendication 9, **caractérisé en ce que** le moteur (24) a des dimensions radiales plus petites que l'élément poussoir (22) de sorte que, dans la position la plus rétractée par rapport au moteur (24) le bord de l'élément poussoir (22) entoure au moins partiellement le moteur (24).

11. Unité selon la revendication 10, **caractérisé en ce qu'**une unité détectrice de position (100, 102, 104) associée au moteur (24) comprend un élément de rotor (100) avec une partie de jupe (102) qui entoure le moteur (24) et qui a des dimensions diamétrales plus petites que l'élément poussoir (22) de sorte que, dans la position la plus rétractée, l'élément poussoir abrite l'élément de rotor (100).
